# EUROPÄISCHE PATENTANMELDUNG

(11) **EP 1 619 188 A1**
(43) Veröffentlichungstag der Anmeldung: **25.01.2006**
(21) Anmeldenummer: 05008949.9
(22) Anmeldetag: 23.04.2005
(51) Int. Cl.: C07D 295/06, C07D 295/14

(54) **Verfahren zur Herstellung von Monoarylpiperazinen**

(30) Priorität: 05.05.2004 DE 102004022765
(71) Anmelder: LANXESS Deutschland GmbH, 51369 Leverkusen (DE)
(72) Erfinder: Scholz, Ulrich ,Dr., 45470 Mühlheim an der Ruhr (DE)

(57) **Zusammenfassung**

Gegenstand der Erfindung ist ein Verfahren zur Herstellung von Arylpiperazinen 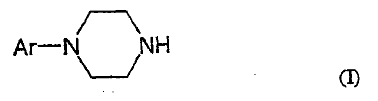 in der
- Ar: für einen mono-, bi- oder tricyclischen aromatischen Rest mit insgesamt 5 bis 18 Ringatomen steht, wobei pro Cyclus keines, ein oder zwei Ringatome ausgewählt ist aus der Gruppe Sauerstoff, Schwefel und Stickstoff und wobei der mono-, bi- oder tricyclische aromatische Rest gegebenenfalls einfach oder mehrfach substituiert ist,
das dadurch gekennzeichnet ist, dass
- Verbindungen der Formel (II)

   Ar―Hal (II)

   in der Ar die vorstehend genannte Bedeutung besitzt und Hal für Chlor, Brom oder Iod steht
- mit Piperazin umsetzt werden,
wobei die Umsetzung
- in Gegenwart von Palladiumkomplexen, die als Liganden Verbindungen der Formel (III) tragen, 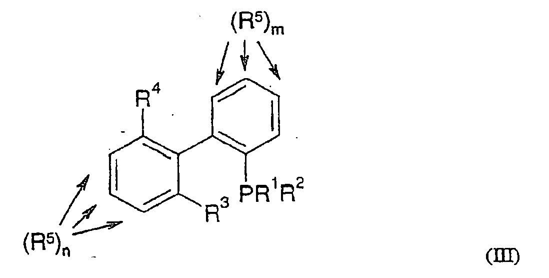
- in Gegenwart von alkalimetallhaltiger Base erfolgt.

## Beschreibung

Gegenstand der Erfindung ist ein Verfahren zur Herstellung von Arylpiperazinen aus den entsprechenden Arylchloriden oder -bromiden und Piperazin unter Verwendung einer Base und eines Katalysators, bestehend aus einem Palladium Salz und einem Bisaryl-dialkylphosphin.

Monoarylpiperazine finden insbesondere als Bausteine zur Herstellung pharmazeutischer Wirkstoffe Verwendung.

Es ist bekannt, Monoarylpiperazine ausgehend von Anilinen durch Reaktion mit aktivierten Diethylenaminen, wie z.B. Di-(chlorethyl)amin herzustellen. Nachteilig an diesen Verfahren ist der Umstand, dass nur wenige hochsubstituierte Aniline kommerziell verfügbar sind und die Wirtschaftlichkeit dieses Verfahrens somit nur für wenige Monoarylpiperazine gegeben ist.

Weiterhin ist aus E. Brenner, R. Schneider, Y. Fort, Tetrahedron 58, (2002), 6913-6924 bekannt, Monoarylpiperazine durch Umsetzung von Chloraromaten und Piperazinen unter Verwendung eines Katalysators herzustellen, wobei der Katalysator aus Nickelacetat und 2,2' -Bipyridin besteht. Nachteilig an diesen Verfahren ist der Umstand, dass die Selektivität von Mono- zu Diarylierung bei diesen Reaktionen für industrielle Zwecke mit 85:15 zu gering ist und darüber hinaus mit 10 mol-% unverhältnismäßig große Mengen des toxischen Nickelacetates eingesetzt werden müssen.

Die Reaktion von substituierten Arylhalogeniden mit Piperazin unter Verwendung eines Palladium-Triarylphosphin-Katalysators wurde erstmalig von Zhao beschrieben (S. Zhao, A.K. Miller, J. Berger, L.A. Flippin, Tetrahedron Letters, 37 (1997), 4463-4466). Dieses Verfahren erfordert jedoch ebenfalls große Katalysatormengen von 2 bis 5 mol-%, um akzeptable chemische Ausbeuten zu erreichen. Weiterhin nachteilig ist der Umstand, dass hohe Selektivitäten bei der Bildung von Monoarylpiperazinen im Vergleich zu Diarylpiperazinen nur erreicht werden können, wenn ein sehr hoher Überschuss von vier bis sechs Äquivalenten an Piperazin zugegeben wird, was sowohl die Aufarbeitung der Reaktion erschwert, wie auch zu höheren Kosten für die Durchführung der Reaktion führt.

Die Selektivität der Reaktion kann durch Verwendung von einfach geschütztem Piperazin in der Reaktion erhöht werden (F. Kerrigan, C. Martin, G.H.Thomas, Tetrahedron Letters, 39 (1998), 2219-2222). Diese Maßnahme bedingt aber durch die Herstellung des geschützten Piperazins sowie die nachträgliche Entfernung der Schutzgruppe zwei unerwünschte zusätzliche Verfahrensschritte, die das Gesamtverfahren unwirtschaftlich machen.

Akzeptable Ausbeuten unter Verwendung geringerer Mengen des Katalysators wurden durch Verwendung eines Palladium-Trisalkylphosphin-Katalysators erreicht (M. Nishiyama, Y. Koie, EP 0 802 173). Nachteilig an diesem Verfahren ist jedoch, dass ein sechsfacher Überschuss an Piperazin eingesetzt werden muss. Weiterhin wird dieses Verfahren unter Einsatz eines hochgradig luftempfindlichen und selbstentzündlichen Phosphins durchgeführt, so dass die Wirtschaftlichkeit des Verfahrens durch zusätzlich erforderliche Sicherheitsmaßnahmen leidet. Weiterhin gelingt dieses Verfahren oft nur bei Einsatz der teuren Arylbromide.

Die Reaktion von Arylchloriden und Piperazin unter Verwendung eines Katalysators bestehend aus luftstabilen N-heterocyclischen Carbenen und einem Palladium-Salz sowie einer starken Alkoholat-Base ist ebenfalls bereits beschrieben worden (S.R. Stauffer, S. Lee, J.P. Stambuli, S.I. Hauk, J.F. Hartwig, Organic Letters 2 (10), 2000, 1423-1426), jedoch wird hier zur Herstellung der Arylpiperazine ausdrücklich erwähnt, dass große Katalysatormengen von 2 mol% notwendig sind. Auch in diesem Verfahren ist ein sehr hoher Überschuss an Piperazin notwendig.

Es bestand demnach weiterhin das Bedürfnis, ein Verfahren bereitzustellen, mit dem eine Vielzahl von Arylhalogeniden mit guten chemischen Ausbeuten wirtschaftlich zu Monoarylpiperazinen umgesetzt werden können.

Es wurde nun ein Verfahren zur Herstellung von Verbindungen der Formel (I) gefunden 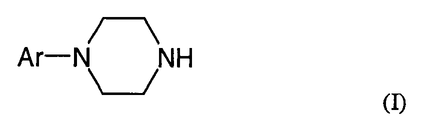 in der
- Ar: für einen mono-, bi- oder tricyclischen aromatischen Rest mit insgesamt 5 bis 18 Ringatomen steht, wobei pro Cyclus keines, ein oder zwei Ringatome ausgewählt ist aus der Gruppe Sauerstoff, Schwefel und Stickstoff und wobei der mono-, bi- oder tricyclische aromatische Rest gegebenenfalls einfach oder mehrfach substituiert ist,
das dadurch gekennzeichnet ist, dass
- Verbindungen der Formel (II)

   Ar―Hal (II)

   in der Ar die vorstehend genannte Bedeutung besitzt und Hal für Chlor, Brom oder Iod steht
- mit Piperazin umsetzt werden,
   wobei die Umsetzung
- in Gegenwart von Palladiumkomplexen, die als Liganden Verbindungen der Formel (III) tragen, 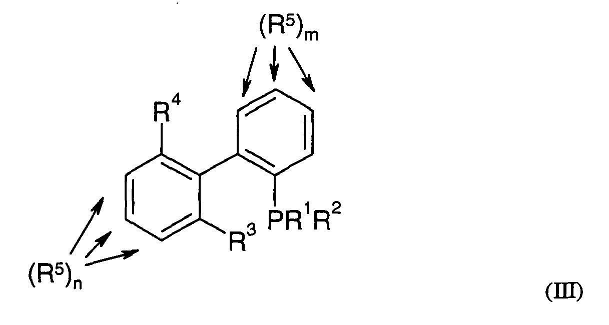 in der
   R¹ und R² jeweils unabhängig voneinander für C₁-C₁₂-Alkyl oder C₅-C₁₁-Arylalkyl stehen
   R³ und R⁴ jeweils unabhängig voneinander für H, C₁-C₆-Alkyl, C₁-C₆-Alkoxy oder Di-C₁-C₆-Alkylamino, bevorzugt für C₁-C₆-Alkyl oder C₁-C₆-Alkoxy stehen,
   die Reste R⁵ jeweils unabhängig voneinander für C₁-C₁₂-Alkyl, C₁-C₁₂-Alkoxy, C₁-C₁₂-Fluoralkyl, C₁-C₁₂-Fluoralkoxy, Fluor, C₄-C₁₀-Aryl oder C₅-C₁₁-Arylalkyl stehen und die Pfeile die möglichen Bindungsstellen zum jeweiligen Arylrest anzeigen und
   n und m jeweils unabhängig voneinander für 0, 1, 2, oder 3 stehen und
- in Gegenwart von alkalimetallhaltiger Base erfolgt.

Der Rahmen der Erfindung umfasst alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Kombination.

**Alkyl** beziehungsweise **Alkoxy** steht jeweils unabhängig für einen geradkettigen, cyclischen, verzweigten oder unverzweigten Alkyl- beziehungsweise Alkoxy-Rest, wobei die genannten Reste gegebenenfalls durch C₁-C₄-Alkoxy-Reste weiter substituiert sein können. Gleiches gilt für den Alkylenteil eines Arylalkylrestes oder den Alkylteil eines Di-Alkylaminorestes.

C₁-C₆-Alkyl steht beispielsweise und bevorzugt für Methyl, Ethyl, n-Propyl, iso-Propyl, n-Butyl, sec.-Butyl, tert.-Butyl, n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, C₁-C₁₂-Alkyl darüber hinaus z.B. für n-Heptyl, n-Octyl, n-Decyl und n-Dodecyl.

C₁-C₁₂-Alkoxy steht beispielsweise und bevorzugt für Methoxy, Ethoxy, n-Propoxy, iso-Propoxy, n-Butoxy, sec.-Butoxy, tert.-Butoxy, n-Pentoxy, cyclo-Hexoxy, cyclo-Pentoxy, n-Hexoxy, C₁-C₁₂-Alkoxy darüber hinaus z.B. für n-Heptoxy, n-Octoxy, n-Decoxy und n-Dodecoxy.

Di-C₁-C₆-Alkylamino steht beispielsweise bevorzugt für N,N-Dimethylamino, N,N-Diethylamino oder N,N-Di-isopropylamino.

Fluoralkyl beziehungsweise Fluoralkoxy steht jeweils unabhängig für einen geradkettigen, cyclischen, verzweigten oder unverzweigten Alkyl-Rest beziehungsweise Alkoxy-Rest, der einfach, mehrfach oder vollständig durch Fluoratome substituiert ist.

Beispielsweise steht C₁-C₁₂-Fluoralkyl für Trifluormethyl, 2,2,2-Trifluorethyl, Pentafluorethyl, Nonafluorbutyl, Heptafluorisopropyl, Perfluoroctyl und Perfluordodecyl.

Beispielsweise steht C₁-C₁₂-Fluoralkoxy für Trifluormethoxy, 2,2,2-Trifluorethoxy, Pentafluorethoxy, Nonafluorbutoxy, Heptafluorisopropoxy, Perfluoroctoxy und Perfluordodecoxy.

Aryl steht jeweils für einen Rest mit 5 bis 10 Gerüstkohlenstoffatomen, in denen heteroaromatischen ein, zwei oder drei Gerüstkohlenstoffatome pro Cyclus, durch Heteroatome, ausgewählt aus der Gruppe Stickstoff, Schwefel oder Sauerstoff, substituiert sein können, oder vorzugsweise für einen carbocyclischen aromatischen Rest mit 6 bis 14 Gerüstkohlenstoffatomen.

Beispiele für mono-, bi- oder tricyclische carbocyclische aromatische Reste mit 6 bis 10 Gerüstkohlenstoffatomen sind Phenyl, Biphenyl, Naphtyl, Phenanthrenyl, Anthracenyl oder Fluorenyl, mono-, bi- oder tricyclische heteroaromatische Reste mit 5 bis 14 Gerüstkohlenstoffatomen in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, sind beispielsweise Pyridinyl, Oxazolyl, Benzofuranyl, Dibenzofuran-yl oder Chinolinyl.

Weiterhin kann der carbocyclische aromatische Rest oder heteroaromatische Rest mit bis zu fünf gleichen oder verschiedenen Substituenten pro Cyclus substituiert sein, die ausgewählt sind aus der Gruppe Fluor, Cyano, C₁-C₁₂-Alkyl, C₁-C₁₂-Fluoralkyl, C₁-C₁₂-Fluoralkoxy, C₁-C₁₂-Alkoxy oder Di(C₁-C₈-alkyl)amino.

**Arylalkyl** bedeutet jeweils unabhängig einen geradkettigen, cyclischen, verzweigten oder unverzweigten Alkyl-Rest nach vorstehender Definition, der einfach, mehrfach oder vollständig durch Aryl-Reste gemäß vorstehender Definition substituiert sein kann.

Im Folgenden werden die bevorzugten Substitutionsmuster für die Formeln (I) und (II) definiert:
- Ar: steht bevorzugt für Aryl nach vorstehender Definition, besonders bevorzugt für einen Phenyl oder Naphthylrest, der nicht, einfach, zweifach oder dreifach mit Resten substituiert ist, die ausgewählt sind aus der Gruppe Nitro, Cyano, Chlor, Fluor, C₁-C₁₂-Alkyl, C₁-C₁₂-Fluoralkyl, C₁-C₁₂-Fluoralkoxy, C₁-C₁₂-Alkoxy, Di(C₁-C₈-alkyl)amino und ganz besonders bevorzugt für einen Phenylrest, der nicht, einfach, zweifach oder dreifach mit Resten substituiert ist, die ausgewählt sind aus der Gruppe Fluor, C₁-C₄-Alkyl, C₁-C₄-Fluoralkyl, C₁-C₄-Fluoralkoxy oder C₁-C₄-Alkoxy.
- Hal: steht bevorzugt für Chlor oder Brom, besonders bevorzugt für Chlor.

Eine besonders bevorzugte Verbindung der Formel (I) ist 4-Trifluormethylphenylpiperazin, eine besonders bevorzugte Verbindung der Formel (II) 4-Trifluormethylchlorbenzol (oft auch als 4-Chlorbenzotrifluorid bezeichnet).

Im Folgenden werden die bevorzugten Substitutionsmuster für Formel (III) definiert:
- R¹und R²: stehen bevorzugt jeweils unabhängig für iso-Propyl, tert-Butyl, Cyclopentyl oder Cyclohexyl, besonders bevorzugt jeweils identisch für vorstehend genannte Reste und ganz besonders bevorzugt jeweils identisch für Cyclohexyl.
- R³ und R⁴: stehen bevorzugt jeweils unabhängig für H, Methyl, Ethyl, iso-Propyl, Methoxy oder N,N-Dimethylamino, besonders bevorzugt für Methyl, Ethyl, iso-Propyl oder Methoxy, ganz besonders bevorzugt jeweils identisch für vorstehend genannte Reste und in bevorzugten Ausführungsformen jeweils identisch für iso-Propyl oder Methoxy.
- n: steht bevorzugt für 0 oder 1,
- m: steht bevorzugt für 0.
- R⁵: steht bevorzugt jeweils unabhängig für Methyl, Ethyl, iso-Propyl und Methoxy.

Besonders bevorzugte Verbindungen der Formel (III) sind [2-(2,4,6-Triisopropylphenyl)-phenyl]-dicyclohexylphosphin und [2-(2,6-Dimethoxyphenyl)-phenyl]-dicyclohexylphosphin.

Die Verbindungen der Formel (III) sind literaturbekannt und beispielsweise gemäß E.R. Strieter, D.G. Blackmond, S.L. Buchwald, Journal of the American Chemical Society, 125, 2003, 13978-13980 herstellbar.

Als Palladiumkomplexe, die als Liganden Verbindungen der Formel (III) tragen, kommen beispielsweise isolierte oder präformierte Palladiumkomplexe enthaltend Verbindungen der Formel (III) oder solche in Betracht, die durch Umsetzung eines Palladiumprecursors mit Verbindungen der Formel (III) im Reaktionsmedium erzeugt werden.

Vorzugsweise werden die für das Verfahren eingesetzten Palladiumkomplexe durch Umsetzung eines Palladiumprecursors mit Verbindungen der Formel (III) im Reaktionsmedium erzeugt.

Geeignete Palladiumprecursoren sind alle Palladiumverbindungen, die mit Verbindungen der Formel (III) unter Ausbildung einer Palladium-Phosphor-Koordination reagieren können.

Bevorzugte Palladiumprecursoren sind: Pd₂(dibenzylidenaceton)₃ oder Allylpalladiumchlorid oder -bromid oder Palladiumverbindungen der Formel (IVa),

PdY¹₂ (IVa)

in der
- Y¹: für ein Anion, bevorzugt für Chlorid, Bromid, Acetat, Propionat, Nitrat, Methansulfonat, Trifluormethansulfonat, Acetylacetonat, Allyl oder Cyclopentadienyl steht,
oder Palladiumverbindungen der Formel (IVb)

PdY²₂L₂ (IVb)

in der
- Y²: für ein Anion, bevorzugt Chlorid, Bromid, Acetat, Methansulfonat oder Tri-fluormethansulfonat, Nonafluorbutansulfonat, Tetrafluoroborat oder Hexafluorophosphat steht und
- L: jeweils für ein Nitril, bevorzugt Acetonitril, Benzonitril oder Benzylnitril, oder ein Olefin, bevorzugt Cyclohexen oder Cycloocten, steht, oder
- L₂: zusammen für ein Diolefin, bevorzugt Norbornadien oder 1,5-Cyclooctadien steht.

Bevorzugt sind als Palladiumprecursoren Palladium(II)acetat und [Pd₂(dba)₃].

Das molare Verhältnis von Palladium zu Verbindungen der Formel (III) kann beispielsweise 1 bis 4 betragen, bevorzugt jedoch 1,5 bis 2,5 und besonders bevorzugt 1,5 bis 2,2, insbesondere genau 2.

Das molare Verhältnis von Palladium zu Verbindungen der Formel (I) kann beispielsweise 0,000001 bis 0,05 betragen, bevorzugt jedoch 0,00001 bis 0,01 und besonders bevorzugt 0,0001 bis 0,001.

Das erfindungsgemäße Verfahren wird in Gegenwart von alkalimetallhaltiger Base durchgeführt. Alkalimetallhaltige Basen sind beispielsweise und bevorzugt Alkalimetall- oder Erdalkalimetall-hydroxide oder -alkanolate, wie beispielsweise Lithium-, Natrium-, Kalium- oder Calciumhydroxid, Natrium- oder Kalium-methanolat, -ethanolat, -n- oder -i-propanolat, -n-, -i-, -s- oder -t-butanolat oder i-Pentanolat; besonders bevorzugt Natrium- oder Kalium-hydroxid oder -tertiärbutanolat und ganz besonders bevorzugt Natriumhydroxid.

Das molare Verhältnis von alkalimetallhaltiger Base zu Verbindungen der Formel (I) kann beispielsweise 1 bis 2,2 betragen, bevorzugt jedoch 1,2 bis 1,6 und besonders bevorzugt 1,4. Größere Mengen sind möglich, jedoch unwirtschaftlich.

Das molare Verhältnis von Piperazin zu Verbindungen der Formel (I) kann beispielsweise 1 bis 3, bevorzugt jedoch 1,5 bis 2 und besonders bevorzugt 1,5. Größere Mengen sind möglich, jedoch unwirtschaftlich.

In einer bevorzugten Ausführungsform wird das Verfahren weiterhin in Gegenwart von organischem Lösungsmittel durchgeführt. Als organische Lösungsmittel eignen sich insbesondere aromatische Kohlenwasserstoffe, wie beispielsweise Benzol, Toluol und Xylole; Amide, wie beispielsweise N,N-Dimethylformamid, N,N-Dimethylacetamid, N-Methyl-formanilid, N-Methylpyrrolidon und N-Methylcaprolactam; Ether, wie beispielsweise Diethylether, Methyl-tert.-butylether, Diisopropylether, Dioxan, Tetrahydrofuran oder Ethylenglykoldimethyl- oder -diethylether; Alkohole, wie beispielsweise Methanol, Ethanol, n- oder i-Propanol, tert.- Butanol, Ethylenglykolmonomethylether, Ethylenglykolmonoethylether, Diethylenglykolmonomethylether, Diethylenglykolmonoethylether, tertiäre Amine, wie beispielsweise Tri-n-butylamin oder Triethylamin oder Gemische solcher organischen Lösungsmittel.

Als organische Lösungsmittel haben sich besonders bewährt: Mischungen von aromatischen Kohlenwasserstoffen und Ethern, wie insbesondere eine Mischung aus Toluol und Tetrahydrofuran, Mischungen von aromatische Kohlenwasserstoffen und Alkoholen wie insbesondere Mischungen aus Toluol und Methanol sowie tertiäre Amine.

Die Reaktionstemperatur kann beispielsweise 30 bis 150°C betragen, bevorzugt 60 bis 150°C, besonders bevorzugt 70 bis 120°C, der Reaktionsdruck 0,5 bis 100 bar, bevorzugt ist Umgebungsdruck.

Auf erfindungsgemäße Weise können Verbindungen der Formel (I) in hoher Reinheit und Ausbeute in einfacher Weise erhalten werden.

Der Vorteil des erfindungsgemäßen Verfahrens liegt insbesondere darin, dass bei geringen Katalysatormengen hohe chemische Ausbeuten an Monoarylpiperazinen erhalten werden können, ohne einen hohen Überschuß an Piperazin verwenden zu müssen.

### Beispiele:

### Beispiele 1 bis 6

### Vergleichsexperiment

Chlorbenzotrifluorid (18,5g, 100 mmol) und Piperazin (12,9g) wurden in 400 ml Xylol vorgelegt und durch Durchleiten von Stickstoff 15 min. bei Raumtemperatur entgast. Trockenes Natrium-tert.butylat (13,5 g, 140mmol) wurde zugegeben und weitere 10 min. entgast. In einem separaten Gefäß wurden Tris-tert.-butylphosphin (81 mg, 0,4 mmol) unter sorgfältigem Ausschluss von Luft und Palladium-dibenzylideneaceton (41 mg, 0,05 mmol) in 10 ml entgastem Tetrahydrofuran unter Stickstoff gerührt. Nach 30 min. wurde diese Katalysatorlösung mithilfe einer Überführungsnadel in den größeren Kolben bei Raumtemperatur eingetropft. Nach vollständiger Zugabe wurde die Reaktion auf 120°C Innentemperatur erwärmt. Nach 8 h ließ man die Reaktion auf 50°C abkühlen und filtrierte vom ausgefallen Feststoff ab. Das Lösungsmittel wurde im Vakuum entfernt und das Rohprodukt durch chromatographische Reinigung an Kieselgel isoliert. Man erhielt 12,4 g (54 mmol, 54% d. Th.) N-(4-Trifluoromethylphenyl)-piperazin.

### Beispiel 1

Chlorbenzotrifluorid (18,5 g, 100 mmol) und Piperazin (12,9g, 150 mmol) wurden in einem Gemisch von 120 ml Toluol und 80 ml Tetrahydrofuran gelöst und durch Durchleiten von Stickstoff 15 min. bei Raumtemperatur entgast. Trockenes Natrium-tert.butylat (13,5 g, 140 mmol) wurde zugegeben und weitere 10 min. entgast. In einem separaten Gefäß wurden [2-(2,6-Dimethoxyphenyl)-phenyl]-dicyclohexylphosphin (41 mg, 0,1 mmol) und Palladium-dibenzylideneaceton (20 mg, 0,025 mmol) und in 10ml entgastem Tetrahydrofuran unter Stickstoff gerührt. Nach 30 min. wurde diese Katalysatorlösung mithilfe einer Überführungsnadel in den größeren Kolben bei Raumtemperatur eingetropft. Nach vollständiger Zugabe wurde die Reaktion auf 90°C Innentemperatur erwärmt. Nach 8 h lies man die Reaktion auf 50°C abkühlen und filtrierte vom ausgefallen Feststoff ab. Das Filtrat wurde mit verdünnter Salzsäure bei pH 3 extrahiert. Die wässrige Phase wurde abgetrennt und mithilfe von Natronlauge auf pH 10 eingestellt. Der ausgefallene weiße Feststoff wurde abfiltriert und im Hochvakuum getrocknet. Man erhielt 21,4 g (93 mmol, 93 % d. Th.) N-(4-Trifluoromethylphenyl)-piperazin. Der Gehalt des Feststoffes an Produkt wurde über quantitatives Protonen-NMR auf >99% festgestellt.

### Beispiel 2

Chlorbenzotrifluorid (18,5 g, 100 mmol) und Piperazin (12,9 g, 150 mmol) wurden in einem Gemisch von 120 ml Toluol und 80 ml Tetrahydrofuran gelöst und durch Durchleiten von Stickstoff 15 min. bei Raumtemperatur entgast. Trockenes Natrium-tert.butylat (13,5 g, 140 mmol) wurde zugegeben und weitere 10min. entgast. In einem separaten Gefäß wurden [2-(2,6-Dimethoxyphenyl)-phenyl]-dicyclohexylphosphin (164 mg, 0,4 mmol) und Palladiumacetat (45 mg, 0,2 mmol) und in 10ml entgastem Tetrahydrofuran unter Stickstoff gerührt. Nach 30 min. wurde diese Katalysatorlösung mithilfe einer Überführungsnadel in den größeren Kolben bei Raumtemperatur eingetropft. Nach vollständiger Zugabe wurde die Reaktion auf 90°C Innentemperatur erwärmt. Nach 8 h lies man die Reaktion auf 50°C abkühlen und filtrierte vom ausgefallen Feststoff ab. Das Filtrat wurde mit verdünnter Salzsäure bei pH 3 extrahiert. Die wässrige Phase wurde abgetrennt und mithilfe von Natronlauge auf pH 10 eingestellt. Der ausgefallene weisse Feststoff wurde abfiltriert und im Hochvakuum getrocknet. Man erhielt 21,8 g (95 mmol, 95 % d. Th.) N-(4-Trifluoromethylphenyl)-piperazin. Der Gehalt des Feststoffes an Produkt wurde über quantitatives Protonen-NMR auf >99% festgestellt.

### Beispiel 3

Chlorbenzotrifluorid (18,5 g, 100 mmol) und Piperazin (12,9 g, 150 mmol) wurden in einem Gemisch von 120 ml Toluol und 80 ml Methanol gelöst und durch Durchleiten von Stickstoff 15 min. bei Raumtemperatur entgast. Natriumhydroxid (5,6 g, 140 mmol) wurde zugegeben und weitere 10 min. entgast. In einem separaten Gefäß wurden [2-(2,4,6-Triisopropylphenyl)-phenyl]-dicyclohexylphosphin (95 mg, 0,2 mmol) und Palladium-dibenzylideneaceton (40 mg, 0,05 mmol) und in 10 ml entgastem Tetrahydrofuran unter Stickstoff gerührt. Nach 30 min. wurde diese Katalysatorlösung mithilfe einer Überführungsnadel in den größeren Kolben bei Raumtemperatur eingetropft. Nach vollständiger Zugabe wurde die Reaktion auf 78°C Innentemperatur erwärmt. Nach 8 h lies man die Reaktion auf 50°C abkühlen und filtrierte vom ausgefallen Feststoff ab. Das Filtrat wurde mit verdünnter Salzsäure bei pH 3 extrahiert. Die wässrige Phase wurde abgetrennt und mithilfe von Natronlauge auf pH 10 eingestellt. Der ausgefallene weiße Feststoff wurde abfiltriert und im Hochvakuum getrocknet. Man erhielt 21,8 g (95 mmol, 95 % d. Th.) N-(4-Trifluoromethylphenyl)-piperazin. Der Gehalt des Feststoffes an Produkt wurde über quantitatives Protonen-NMR auf >99% festgestellt.

### Beispiel 4

Chlorbenzotrifluorid (18,5 g, 100 mmol) und Piperazin (12,9 g, 150 mmol) wurden in 200 ml Tri-n-butylamin gelöst und durch Durchleiten von Stickstoff 15 min. bei Raumtemperatur entgast. Trockenes Natrium-tert.butylat (13,5 g, 140 mmol) wurde zugegeben und weitere 10 min. entgast. In einem separaten Gefäß wurden [2-(2,6-Dimethoxyphenyl)-phenyl]-dicyclohexylphosphin (41 mg, 0,1 mmol) und Palladium-dibenzylideneaceton (20 mg, 0,025 mmol) und in 5ml entgastem Tetrahydrofuran unter Stickstoff gerührt. Nach 30 min. wurde diese Katalysatorlösung mithilfe einer Überführungsnadel in den größeren Kolben bei Raumtemperatur eingetropft. Nach vollständiger Zugabe wurde die Reaktion auf 110°C Innentemperatur erwärmt. Nach 8 h lies man die Reaktion auf 50°C abkühlen und filtrierte vom ausgefallen Feststoff ab. Das Filtrat wurde im Vakuum eingeengt und mit verdünnter Salzsäure bei pH 3 extrahiert. Die wässrige Phase wurde abgetrennt und mithilfe von Natronlauge auf pH 10 eingestellt. Der ausgefallene weiße Feststoff wurde abfiltriert und im Hochvakuum getrocknet. Man erhielt 22,1 g (96 mmol, 96 % d. Th.) N-(4-Trifluoromethylphenyl)-piperazin. Der Gehalt des Feststoffes an Produkt wurde über quantitatives Protonen-NMR auf >99% festgestellt.

### Beispiel 5

Brombenzotrifluorid (22,5 g, 100 mmol) und Piperazin (12,9 g, 150 mmol) wurden in 200 ml Toluol gelöst und durch Durchleiten von Stickstoff 15 min. bei Raumtemperatur entgast. Trockenes Natrium-tert.butylat (13,5 g, 140 mmol) wurde zugegeben und weitere 10 min. entgast. In einem separaten Gefäß wurden [2-(2,4,6-Triisopropylphenyl)-phenyl]-dicyclohexylphosphin (95 mg, 0,2 mmol) und Palladiumacetat (23 mg, 0,1 mmol) und in 10 ml entgastem Tetrahydrofuran unter Stickstoff gerührt. Nach 30 min. wurde diese Katalysatorlösung mithilfe einer Überführungsnadel in den größeren Kolben bei Raumtemperatur eingetropft. Nach vollständiger Zugabe wurde die Reaktion auf 110°C Innentemperatur erwärmt. Nach 8 h lies man die Reaktion auf 50°C abkühlen und filtrierte vom ausgefallen Feststoff ab. Das Filtrat wurde mit verdünnter Salzsäure bei pH 3 extrahiert. Die wässrige Phase wurde abgetrennt und mithilfe von Natronlauge auf pH 10 eingestellt. Der ausgefallene weiße Feststoff wurde abfiltriert und im Hochvakuum getrocknet. Man erhielt 22,1 g (96 mmol, 96 % d. Th.) N-(4-Trifluoromethylphenyl)-piperazin. Der Gehalt des Feststoffes an Produkt wurde über quantitatives Protonen-NMR auf >99% festgestellt.

### Beispiel 6

4-Cyanochlorbenzol (13,8 g, 100 mmol) und Piperazin (12,9 g, 150 mmol) wurden in einem Gemisch von 120 ml Toluol und 80 ml Tetrahydrofuran gelöst und durch Durchleiten von Stickstoff 15 min. bei Raumtemperatur entgast. Trockenes Natrium-tert.butylat (13,5 g, 140 mmol) wurde zugegeben und weitere 10 min. entgast. In einem separaten Gefäß wurden [2-(2,4,6-Triisopropylphenyl)-phenyl]-dicyclohexylphosphin (95 mg, 0,2 mmol) und Palladium-dibenzylideneaceton (40 mg, 0,05 mmol) in 10 ml entgastem Tetrahydrofuran unter Stickstoff gerührt. Nach 30 min. wurde diese Katalysatorlösung mithilfe einer Überführungsnadel in den größeren Kolben bei Raumtemperatur eingetropft. Nach vollständiger Zugabe wurde die Reaktion auf 90°C Innentemperatur erwärmt. Nach 8h lies man die Reaktion auf 50°C abkühlen und filtrierte vom ausgefallen Feststoff ab. Das Filtrat wurde mit verdünnter Salzsäure bei pH 3 extrahiert. Die wässrige Phase wurde abgetrennt und mithilfe von Natronlauge auf pH 10 eingestellt. Der ausgefallene weiße Feststoff wurde abfiltriert und im Hochvakuum getrocknet. Man erhielt 17,8 g (95 mmol, 95 % d.Th.) N-(4-Cyanophenyl)-piperazin. Der Gehalt des Feststoffes an Produkt wurde über quantitatives Protonen-NMR auf >99% festgestellt.

## Patentansprüche

1. Verfahren zur Herstellung von Verbindungen der Formel (I), 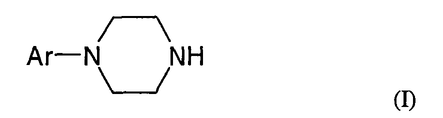 in der
Ar für einen mono-, bi- oder tricyclischen aromatischen Rest mit insgesamt 5 bis 18 Ringatomen steht, wobei pro Cyclus keines oder ein Ringatom ausgewählt ist aus der Gruppe Sauerstoff, Schwefel und Stickstoff und wobei der mono-, bi- oder tricyclische aromatische Rest gegebenenfalls einfach oder mehrfach substituiert ist,
**dadurch gekennzeichnet, dass**
• Verbindungen der Formel (II)
Ar―Hal (II)
in der Ar die vorstehend genannte Bedeutung besitzt und Hal für Chlor, Brom oder Iod steht
• mit Piperazin umsetzt werden,
wobei die Umsetzung
• in Gegenwart von Palladiumkomplexen, die als Liganden Verbindungen der Formel (III) tragen, 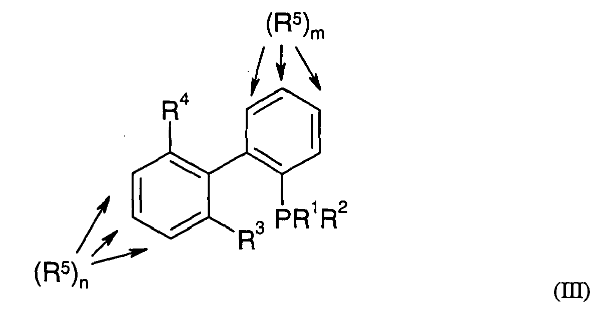 in der
R¹ und R² jeweils unabhängig voneinander für C₁-C₁₂-Alkyl oder C₅-C₁₁-Arylalkyl stehen
R³ und R⁴ jeweils unabhängig voneinander für H, C₁-C₆-Alkyl, C₁-C₆-Alkoxy oder Di-C₁-C₆-Alkylamino stehen,
die Reste R⁵ jeweils unabhängig voneinander für C₁-C₁₂-Alkyl, C₁-C₁₂-Alkoxy, C₁-C₁₂-Fluoralkyl, C₁-C₁₂-Fluoralkoxy, Fluor, C₄-C₁₀-Aryl oder C₅-C₁₁-Arylalkyl stehen und die Pfeile die möglichen Bindungsstellen zum jeweiligen Arylrest anzeigen und
n und m jeweils unabhängig voneinander für 0, 1, 2, oder 3 stehen und
• in Gegenwart von alkalimetallhaltiger Base erfolgt.

2. Verfahren nach Anspruch 1, **dadurch gekennzeichnet, dass** als Palladiumkomplexe, die als Liganden Verbindungen der Formel (III) tragen, solche eingesetzt werden, die durch Umsetzung eines Palladiumprecursors mit Verbindungen der Formel (III) im Reaktionsmedium erzeugt werden.

3. Verfahren nach Anspruch 1 oder 2, **dadurch gekennzeichnet, dass** das molare Verhältnis von Palladium zu Verbindungen der Formel (III) 1 bis 4 beträgt.

4. Verfahren nach Anspruch 1, 2 oder 3, **dadurch gekennzeichnet, dass** das molare Verhältnis von Palladium zu Verbindungen der Formel (I) 0,000001 bis 0,05 beträgt.

5. Verfahren nach Anspruch 1, 2, 3 oder 4, **dadurch gekennzeichnet, dass** als alkalimetallhaltige Basen Alkalimetall- oder Erdalkalimetall-hydroxide oder -alkanolate eingesetzt werden.

6. Verfahren nach Anspruch 1, 2, 3, 4 oder 5, **dadurch gekennzeichnet, dass** das molare Verhältnis von Piperazin zu Verbindungen der Formel (I) 1,2 bis 1,8 beträgt.

7. Verfahren nach Anspruch 1, 2, 3, 4, 5 oder 6, **dadurch gekennzeichnet, dass** das Verfahren weiterhin in Gegenwart von organischem Lösungsmittel durchgeführt wird.

8. Verfahren nach Anspruch 7, **dadurch gekennzeichnet, dass** als organische Lösungsmittel aromatische Kohlenwasserstoffe, Amide, Ether, Alkohole, tertiäre Amine oder Gemische solcher organischen Lösungsmittel eingesetzt werden.
